# EUROPEAN PATENT SPECIFICATION

(11) **EP 1 561 477 B1**
(45) Date of publication and mention of the grant of the patent: **06.05.2009**
(21) Application number: 05100695.5
(22) Date of filing: 01.02.2005
(51) Int. Cl.: A61L 9/04, A61L 9/12

(54) **Vehicle passenger compartment air freshener dispenser**
Luftverbesserer für Fahrzeuginnenraum
Déodorisateur d'air pour l' intérieur des automobiles

(30) Priority: 02.02.2004 IT MI20040160
(43) Date of publication of application: 10.08.2005
(73) Proprietor: Petronas Lubricants Italy S.p.A., Villastellone (IT)
(72) Inventor: Bonanno, Giovanni, 20063, Cernusco sul Naviglio (IT); Cossettini, Paola, 20066, Melzo (IT)
(74) Representative: Jorio, Paolo

(56) References cited:
- EP-A- 0 495 631
- WO-A-02/09772
- US-A- 5 178 327
- US-A- 5 220 636
- PATENT ABSTRACTS OF JAPAN vol. 2000, no. 05, 14 September 2000 (2000-09-14) & JP 2000 062511 A (NAPOLEX CO), 29 February 2000 (2000-02-29)

## Description

The present invention relates to a vehicle passenger compartment air freshener dispenser.

Air freshener dispensers are used in vehicles to impart a pleasant fragrance to the passenger compartment. Given the relatively small size of the passenger compartment and the different temperature conditions in which the vehicle operates, automotive air fresheners call for careful design and controlled freshener release. To meet varying demand by motorists, various types of dispensers have been devised, and which can be divided into two groups : a first in which the freshener is diluted in a liquid, and a second in which the freshener is used to impregnate solid elements. The second group in turn comprises dispensers in which the solid element comprises a single, relatively large element, and dispensers employing relatively small granules. Granules have the advantage of permitting highly effective controlled release by the dispenser, particularly dispensers in which air is channeled through the granules. Dispensers of this sort come in various fragrances, depending on the fragrance with which the granules are impregnated, but have the drawback of requiring that the fragrance be somehow indicated on the container to associate a given container with a given fragrance.

WO 02/09772 discloses an air freshener dispenser that is rather complicated and comprises a cartridge for containing a substances impregnated with a fragrance. The dispenser may provided with a transparent wall to see the cartridge. However, the cartridge is provided with a barrier and information have some how to be written on the cartridge.

EP 495,631 discloses air freshener dispenser comprising a container for containing a gel mass impregnated with a fragrance and having an opening closed by a seal that is removed totally or partially to let the fragrance to flow into the environment. The gel mass is colored so as to contrast with the wall and to indicate the reduction in size of the gel mass. This air freshener dispenser gives information of the consumption of gel mass only and not to the type of fragrance. Furthermore, the information is given only when the seal is peeled away.

US 5,178,327 discloses an air freshener dispenser comprising a container and a cartridge rotatable inside the container so as to expose a solid substance impregnated with fragrance in correspondence of an aperture of the container. This dispenser allow to see only the fragrance arranged in corresponding of the aperture and do not give any kind of information.

All the prior art documents listed above suffer from the above-identified drawbacks.

It is an object of the present invention to provide a granule air freshener dispenser designed to eliminate the aforementioned drawback.

According to the present invention, there is provided an air freshener dispenser for a vehicle passenger compartment, the dispenser comprising a container of scented granules, and a fastening device for fixing the container to the front of an air outlet of the passenger compartment; the container having openings allowing airflow from the air outlet to flow partly through the scented granules into the passenger compartment; said scented granules being available in different fragrances and colored to indicate the relative fragrance and to impart an attractive appearance to the dispenser; the container comprising a rear wall and a front wall facing each other and having, respectively, a first and a second portion having said openings to allow the airflow from the air outlet to flow partly through the container and the scented granules into the passenger compartment; the container comprising a number of compartments, each containing said scented granules and being movable selectively into position between the first and the second portion; the container comprising a first part comprising said first and said second portion; and a second part movable with respect to the first part and defining said number of compartments; said first part comprising a shell; and said second part comprising a tub, which is mounted to rotate with respect to the shell about a given axis and is made of transparent material.

Once the dispenser runs out, the user, if pleased with the fragrance, can therefore purchase another simply on the basis of photographic memory, by simply selecting a dispenser with scented granules of the same colour. Moreover, the container need not be of a particular colour to attract the consumer, this function being performed by the coloured, scented granules.

A preferred, non-limiting embodiment of the present invention will be described by way of example with reference to the accompanying drawings, in which:
Figure 1 shows a front view in perspective of an air freshener dispenser in accordance with the present invention and fitted to an air outlet of a vehicle;
Figure 2 shows a rear view in perspective of the Figure 1 dispenser;
Figure 3 shows an exploded view of the Figure 1 dispenser.

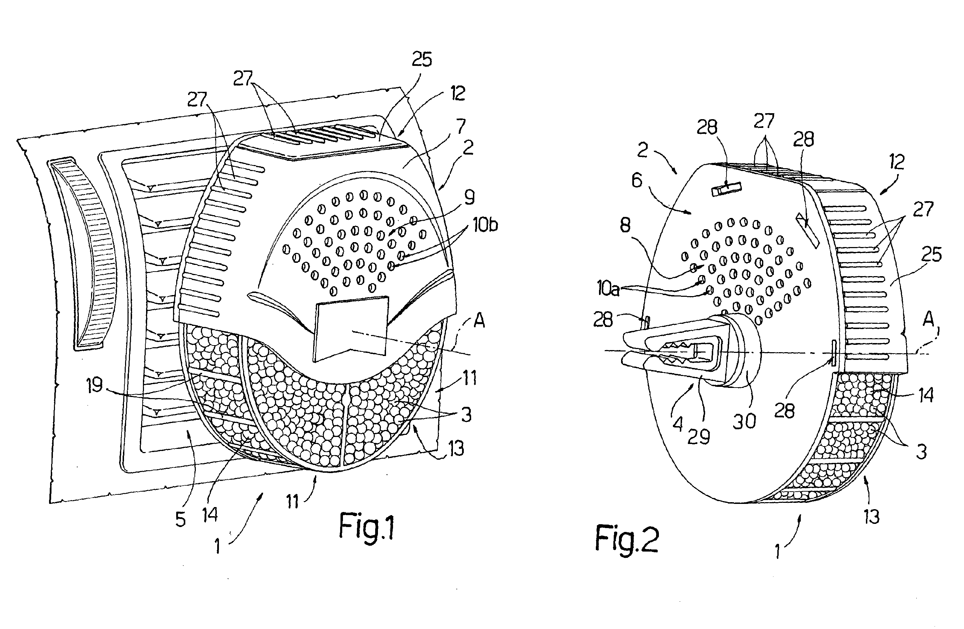

Number 1 in Figure 1 indicates as a whole an air freshener dispenser for a vehicle passenger compartment (not shown). Dispenser 1 comprises a container 2 of scented granules 3, and a fastening device 4 (Figure 2) for selectively fixing container 2 to an air outlet 5 (Figure 1) of the passenger compartment. With reference to Figures 1 and 2, container 2 comprises a rear wall 6 and a front wall 7 facing each other and having, respectively, a first and a second portion 8 and 9 with respective openings 10a and 10b enabling airflow from air outlet 5 to flow partly through container 2 and scented granules 3 into the passenger compartment.

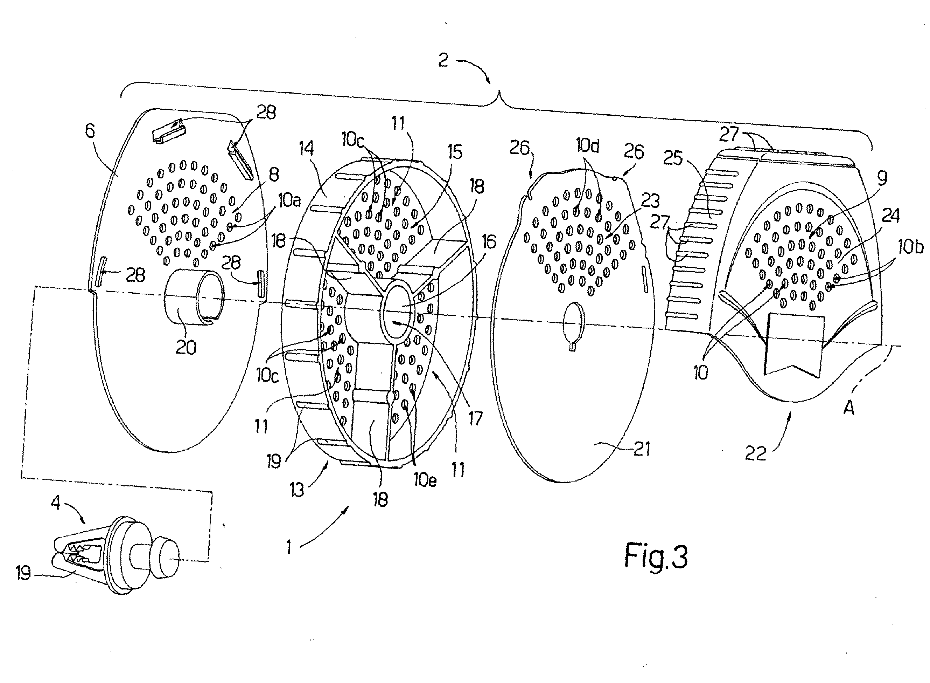

Container 2 comprises a number of compartments 11, each of which contains scented granules 3 and is movable selectively into position between first and second portion 8 and 9. In other words, container 2 comprises a first part comprising first and second portion 8 and 9; and a second part movable with respect to the first part and defining compartments 11. The first part is substantially defined by a shell 12, and the second part is defined by a tub 13, which, as shown more clearly in Figure 3, is fitted to rotate with respect to shell 12 about a given axis A, and comprises an outer cylindrical wall 14, an annular back wall 15, an inner cylindrical wall 16 defining a through hole 17, and a number of radial walls 18 extending from wall 14 to wall 16 and integral with annular wall 15 to define compartments 11. Annular wall 15 of each compartment 11 comprises a number of openings 10c arranged and sized to line up with openings 10a in rear wall 6. The outer face of outer cylindrical wall 14 has ridges 19, parallel to axis A, for firm grip and easy rotation of tub 13.

Shell 12 comprises component parts assembled as shown in Figure 3. A first component part is defined by wall 6, and by a hub 20 integral with wall 6 and extending coaxially with axis A. Shell 12 also comprises an annular disk 21 and a half-shell 22. Disk 21 extends perpendicularly to axis A, and comprises a portion 23 with openings 10d. Half-shell 22 comprises a first wall 24 perpendicular to axis A; and a second wall 25 parallel to axis A. First wall 24 defines, with disk 21, front wall 7 of container 2, extends over an area substantially equal to portion 9 and to a portion about axis A, and comprises openings 10b. Along its outer edge, disk 21 has locators 26 which engage locators on half-shell 22 to align openings 10d perfectly with openings 10b. Wall 24 also has a projection (not shown) which engages hub 20. Wall 25 of half-shell 22 has ridges 27, parallel to axis A, for firm grip by the user's hand; and tabs (not shown) which engage seats 28 in wall 6 to connect half-shell 22 to wall 6.

When container 2 is assembled, tub 13 is supported for rotation by hub 20 engaging hole 17. That is, tub 13 is gripped between walls 6 and 7 of shell 12, while permitting rotation of tub 13. Compartments 11 are identical, and each is substantially the same size, perpendicular to axis A, as portions 8 and 9, so that the airflow into the compartment 11 aligned with portions 8 and 9 blows through all the granules 3 inside compartment 11. Tub 13 can be rotated with respect to shell 12 one-handedly, by placing the thumb on tub 13 and the fingers on wall 25 of shell 12. Ridges 19 and 27 increase friction and improve grip between the moving parts.

Rotation of the tub with respect to the shell moves a compartment 11, containing used-up granules 3, out of alignment with portions 8 and 9, and moves another compartment 11, containing fresh granules 3, into alignment with portions 8 and 9. Rotation of tub 13, particularly by small amounts, also provides for regulating freshener inflow into the passenger compartment by setting openings 10c in tub 13 to a position of partial or full alignment with openings 10a in wall 6. Openings 10c and 10a are shaped and arranged to permit full alignment, to maximize airflow into container 2 and scented granules 3; total misalignment, to prevent airflow through scented granules 3; and an infinite number of intermediate positions.

With reference to Figure 2, fastening device 4 comprises a gripper 29, which clicks inside a pocket 30 formed on the outer face of wall 6, extends along axis A, and rotates about axis A with respect to pocket 30.

Tub 13 and disk 21 are made of transparent material, preferably PCTA (polycyclohexene dimethylen terephthalate). More generally, tub 13 and disk 21 are made of material in the PET-base polyester copolymer group. Only the specific materials indicated for the container give satisfactory results in terms of appearance and performance. Other materials have been found to result in dulling and rapid deterioration of the transparency of the container.

Granules 3 are impregnated with perfume essence to impart a given fragrance, and at the same time are coloured, each fragrance being assigned a specific colour.

Compartments 11 are all filled with granules 3 of the same fragrance and colour, or each compartment 11 is filled with granules 3 of a different fragrance and colour from granules 3 in the adjacent compartment 11.

In a variation not shown, front wall 7 is fully transparent, so that portion 9 with the openings is also transparent, and the compartment 11 in use and the granules 3 inside it are visible. This solution is particularly advantageous when each compartment 11 contains granules 3 of a respective fragrance and colour, so as to associate a colour with the fragrance in use.

Dispenser 1 according to the present invention has various advantages, such as associating a given colour of the granules with a given fragrance, the possibility of adjusting the strength of the fragrance, the ease with which this can be done one-handedly (particularly important in the case of a device fitted inside a vehicle passenger compartment), and the straightforward construction design of the device which makes it particularly cheap to produce.

## Claims

1. An air freshener dispenser for a vehicle passenger compartment, the dispenser (1) comprising a container (2) of scented granules (3), and a fastening device (4) for fixing the container (2) to the front of an air outlet (5) of the passenger compartment; the container (2) having openings (10a, 10b) allowing airflow from the air outlet (5) to flow partly through the scented granules (3) into the passenger compartment; said scented granules (3) being available in different fragrances and colored to indicate the relative fragrance and to impart an attractive appearance to the dispenser (1); the container (2) comprising a rear wall (6) and a front wall (7) facing each other and having, respectively, a first and a second portion (8, 9) having said openings (10a, 10b) to allow the airflow from the air outlet (5) to flow partly through the container (2) and the scented granules (3) into the passenger compartment; the container (2) comprising a number of compartments (11), each containing said scented granules (3) and being movable selectively into position between the first and the second portion (8, 9); the container (2) comprising a first part comprising said first and said second portion (8, 9); and a second part movable with respect to the first part and defining said number of compartments (11); said first part comprising a shell (12); and said second part comprising a tub (13), which is mounted to rotate with respect to the shell (12) about a given axis (A) and is made of transparent material.

2. A dispenser as claimed in Claim 1, **characterized in that** said front wall (7) is at least partly transparent.

3. A dispenser as claimed in Claim 1 or 2, **characterized in that** said tub (13) comprises an outer cylindrical wall (14); a back wall (15) having openings (10c); an inner cylindrical wall (15); and a number of radial walls (18) extending from the outer cylindrical wall (14) to the inner cylindrical wall (16) to define said compartments (11).

4. A dispenser as claimed in Claim 3, **characterized in that** said compartments (11) are identical.

5. A dispenser as claimed in Claim 4, **characterized in that** the first and second portion (8, 9) are substantially the same size as each of said compartments (11) in a plane perpendicular to said axis (A).

6. A dispenser as claimed in any one of Claims 3 to 5, **characterized in that** said openings (10c) in the back wall (15) can be partly and totally aligned with the openings (10a) in the rear wall (6), depending on the position of the tub (13) with respect to the rear wall (6), so as to regulate airflow through the scented granules (3).

7. A dispenser as claimed in any one of Claims 2 to 6, **characterized in that** said shell (12) and said tub (13) comprise respective walls (25, 14), substantially parallel to said axis (A), treated in such a manner as to increase friction in a direction crosswise to said axis (A).

8. A dispenser as claimed in Claim 7, **characterized in that** said walls (25, 14) comprise ridges parallel to said axis (A).

9. A dispenser as claimed in any one of the foregoing Claims, **characterized in that** said transparent material is selected from the PET-based polyester copolymer group.

10. A dispenser as claimed in any one of the foregoing Claims, **characterized in that** said transparent material is PCTA (polycyclohexene dimethylen terephthalate).

## Patentansprüche

1. Lufterfrischer-Spender für einen Fahrzeuginnenraum, wobei der Spender (1) umfasst:
einen Behälter (2) mit parfümiertem Granulat (3), und
eine Befestigungsvorrichtung (4) zum Befestigen des Behälters (2) an der Vorderseite einer Luftaustrittsöffnung (5) des Fahrzeuginnenraums;
wobei der Behälter (2) Öffnungen (10a, 10b) aufweist, die ermöglichen, dass ein Luftstrom von der Luftaustrittsöffnung (5) teilweise durch das parfümierte Granulat (3) in den Fahrzeuginnenraum strömt;
wobei das parfümierte Granulat (3) in unterschiedlichen Düften verfügbar ist und gefärbt ist, um den jeweiligen Duft zu kennzeichnen und dem Spender (1) ein ansprechendes Aussehen zu verleihen;
wobei der Behälter (2) eine Rückwand (6) und eine Vorderwand (7) aufweist, die einander gegenüberliegen und jeweils einen ersten und zweiten Abschnitt (8, 9) aufweisen, die die Ausnehmungen (10a, 10b) umfassen, um zu ermöglichen, dass der Luftstrom von der Luftaustrittsöffnung (5) teilweise durch den Behälter (2) und das parfümierte Granulat (3) in den Fahrzeuginnenraum strömt;
wobei der Behälter (2) eine Vielzahl von Kammern (11) aufweist, von denen jede das parfümierte Granulat (3) fasst und wahlweise in eine Position zwischen dem ersten und zweiten Abschnitt (8, 9) schwenkbar ist;
wobei der Behälter (2) umfasst:
ein erstes Teil, das den ersten und zweiten Abschnitt (8, 9) aufweist; und
ein zweites Teil, das bezüglich des ersten Teils schwenkbar ist und die Anzahl der Kammern (11) definiert;
wobei das erste Teil ein Gehäuse (12) aufweist; und das zweite Teil eine Wanne (13) aufweist, die derart angebracht ist, dass sie bezüglich des Gehäuses (12) um eine gegebene Achse (A) verschwenkbar ist, und die aus transparentem Material gefertigt ist.

2. Spender nach Anspruch 1,
**dadurch gekennzeichnet, dass** die Vorderwand (7) zumindest teilweise transparent ist.

3. Spender nach Anspruch 1 oder 2,
**dadurch gekennzeichnet, dass** die Wanne (13) umfasst:
eine äußere zylinderförmige Wand (14);
eine Hinterwand (15), die Öffnungen (10c) aufweist;
eine innere zylinderförmige Wand (16); und
eine Mehrzahl von radialen Wänden (18), die sich von der äußeren zylinderförmigen Wand (14) zu der inneren zylinderförmigen Wand (16) erstrecken, um die Kammern (11) zu definieren.

4. Spender nach Anspruch 3,
**dadurch gekennzeichnet, dass** die Kammern (11) identisch sind.

5. Spender nach Anspruch 4,
**dadurch gekennzeichnet, dass** der erste und zweite Abschnitt (8, 9) in einer Ebene senkrecht zur Achse (A) im Wesentlichen die gleiche Größe aufweisen wie jede der Kammern (11).

6. Spender nach einem der Ansprüche 3 bis 5,
**dadurch gekennzeichnet, dass** abhängig von der Position der Wanne (13) bezüglich der Rückwand (6) die Öffnungen (10c) in der Hinterwand (15) teilweise und vollständig zu den Öffnungen (10a) in der Rückwand (6) ausrichtbar sind, um den Luftstrom durch das parfümierte Granulat (3) zu regulieren.

7. Spender nach einem der Ansprüche 2 bis 6,
**dadurch gekennzeichnet, dass** das Gehäuse (12) und die Wanne (13) jeweils zur Achse (A) im Wesentlichen parallele Wände (25, 14) aufweisen, die derart behandelt sind, dass diese die Reibung in Richtung quer zur Achse (A) erhöhen.

8. Spender nach Anspruch 7,
**dadurch gekennzeichnet, dass** die Wände (25, 14) zur Achse (A) parallele Erhöhungen aufweisen.

9. Spender nach einem der vorangehenden Ansprüche,
**dadurch gekennzeichnet, dass** das transparente Material aus der PET-basierten Polyester-Copolymer-Gruppe ausgewählt ist.

10. Spender nach einem der vorangehenden Ansprüche,
**dadurch gekennzeichnet, dass** das durchsichtige Material PCTA (Polycyclohexen-Dimethylen-Terephthalat) ist.

## Revendications

1. Distributeur de purificateur d'air pour un compartiment de passager de véhicule, le distributeur (1) comprenant un récipient (2) de granulés parfumés (3) et un dispositif de fixation (4) pour fixer le récipient (2) sur l'avant d'une sortie d'air (5) du compartiment de passager ; le récipient (2) ayant des ouvertures (10a, 10b) permettant l'écoulement d'air à partir de la sortie d'air (5) pour s'écouler partiellement en passant par les granulés parfumés (3) dans le compartiment de passager ; lesdits granulés parfumés (3) étant disponibles dans différentes fragrances et colorés pour indiquer la fragrance relative et pour communiquer une apparence attractive au distributeur (1) ; le récipient (2) comprenant une paroi arrière (6) et une paroi avant (7) se faisant face et ayant, respectivement, une première et une seconde partie (8, 9) ayant lesdites ouvertures (10a, 10b) pour permettre l'écoulement d'air à partir de la sortie d'air (5) afin de s'écouler partiellement en passant par le récipient (2) et par les granulés parfumés (3) à l'intérieur du compartiment de passager ; le récipient (2) comprenant un certain nombre de compartiments (11), chacun contenant lesdits granulés parfumés (3) et étant mobile sélectivement dans une position entre la première et la seconde portions (8, 9) ; le récipient (2) comprenant une première partie comprenant ladite première et ladite seconde portions (8, 9) ; et une seconde partie mobile par rapport à la première partie et définissant ledit nombre de compartiments (11) ; ladite première partie comprenant une coque (12) ; et ladite seconde partie comprenant un bac (13) qui est monté pour tourner par rapport à la coque (12) autour d'un axe (A) donné et est réalisée avec un matériau transparent.

2. Distributeur selon la revendication 1, **caractérisé en ce que** ladite paroi avant (7) est au moins partiellement transparente.

3. Distributeur selon la revendication 1 ou 2, **caractérisé en ce que** ledit bac (13) comprend une paroi cylindrique externe (14) ; une paroi de fond (15) ayant des ouvertures (10c) ; une paroi cylindrique interne (16) ; et un certain nombre de parois radiales (18) s'étendant à partir de la paroi cylindrique externe (14) jusqu'à la paroi cylindrique interne (16) pour définir lesdits compartiments (11).

4. Distributeur selon la revendication 3, **caractérisé en ce que** lesdits compartiments (11) sont identiques.

5. Distributeur selon la revendication 4, **caractérisé en ce que** les première et seconde portions (8, 9) sont sensiblement de la même taille que chacun desdits compartiments (11) dans un plan perpendiculaire audit axe (A).

6. Distributeur selon l'une quelconque des revendications 3 à 5, **caractérisé en ce que** lesdites ouvertures (10c) dans la paroi de fond (15) peuvent être partiellement ou totalement alignées avec les ouvertures (10a) dans la paroi arrière (6) en fonction de la position de la cuve (13) par rapport à la paroi arrière (6) afin de réguler l'écoulement d'air sur les granulés parfumés (3).

7. Distributeur selon l'une quelconque des revendications 2 à 6, **caractérisé en ce que** ladite coque (12) et ledit bac (13) comprennent des parois (25, 14) respectives, sensiblement parallèles audit axe (A), traitées afin d'augmenter le frottement dans une direction transversale par rapport audit axe (A).

8. Distributeur selon la revendication 7, **caractérisé en ce que** lesdites parois (25, 14) comprennent des saillies parallèles audit axe (A).

9. Distributeur selon l'une quelconque des revendications précédentes, **caractérisé en ce que** ledit matériau transparent est choisi parmi le groupe de copolymères polyesters à base de PET.

10. Distributeur selon l'une quelconque des revendications précédentes, **caractérisé en ce que** ledit matériau transparent est le PCTA (polycyclohexylène diméthylène téréphtalate).
